# EUROPEAN PATENT SPECIFICATION

(11) **EP 4 272 786 B1**
(45) Date of publication and mention of the grant of the patent: **22.07.2026**
(21) Application number: 21915804.5
(22) Date of filing: 29.12.2021
(51) Int. Cl.: A61M 5/42, A61M 5/32, A61M 5/31

(54) **PORTABLE LIGHT-GUIDED INJECTION DEVICE**
TRAGBARE LICHTGESTEUERTE INJEKTIONSVORRICHTUNG
DISPOSITIF D'INJECTION PORTATIF GUIDÉ PAR LA LUMIÈRE

(30) Priority: 29.12.2020 KR 20200186760
(43) Date of publication of application: 08.11.2023
(73) Proprietor: Solmedix Co., Ltd., Mapo-gu Seoul (KR)
(72) Inventor: CHUN, Kwon Soo, Bucheon-si Gyeonggi-do 14581 (KR); KANG, Ho Chul, Goyang-si Gyeonggi-do 10450 (KR); YANG, Inchul, Sejong-si 30126 (KR)
(74) Representative: Roos, Peter
(86) International application number: PCT/KR2021/020110
(87) International publication number: WO 2022/146006

(56) References cited:
- CN-A- 108 245 740
- JP-A- 2009 542 419
- KR-A- 20170 005 269
- KR-A- 20170 051 602
- KR-A- 20190 049 097
- KR-B1- 101 699 229
- US-A1- 2003 009 136
- US-A1- 2014 315 165
- US-A1- 2018 185 591
- WONJAE CHA ET AL: "Development of a device for real-time light-guided vocal fold injection: A preliminary report : Real-Time Light-Guided Vocal Fold Injection", THE LARYNGOSCOPE, vol. 126, no. 4, 25 April 2016 (2016-04-25), United States, pages 936 - 940, XP055374123, ISSN: 0023-852X, DOI: 10.1002/lary.25661

## Description

### TECHNICAL FIELD

This application pertains to a portable device for light-guided injections.

### BACKGROUND

Vocal cord paralysis is a condition that occurs due to recurrent laryngeal nerve damage, which can be caused by various factors, including surgical procedures (such as those involving the thyroid, lung, esophagus, heart, head and neck, spine, and brain), cancer invasion (such as thyroid cancer, lung cancer, esophageal cancer, and head and neck cancer), and traumatic incidents (such as traffic accident).

Recently, there has been a rise in the frequency of surgeries and cancers that are recognized as potential causes of vocal cord paralysis. Therefore, many patients suffer from complications such as voice disorders, dysphagia, and aspiration pneumonia.

When vocal cord paralysis results in a less severe voice disorder, it can be effectively managed through voice therapy or rehabilitation. However, in many cases, surgical treatment is necessary to address the condition.

The primary surgical treatment options for vocal cord paralysis are centered on procedures performed under general anesthesia or invasive surgeries that involve external skin incisions, such as thyroid cartilage plastic surgery or arytenoid adduction.

Recently, there has been growing attention on a simple and effective non-invasive surgical method called vocal cord injection, thanks to the advancements in bio-injection materials and the improvements in material stability.

Vocal cord injection is primarily employed as a primary treatment for unilateral vocal cord paralysis and spasmodic dysphonia. However, it still has certain drawbacks concerning the ease and precision of the procedure.

The precise positioning of percutaneous vocal cord injection is currently reliant on the operator's skills and experience, as there is no technology or equipment available to assist in this regard.

Acquiring proficiency in percutaneous vocal cord injection usually requires extensive experience working with numerous patients. The learning curve associated with this procedure is relatively steep, resulting in a high entry barrier for individuals with limited experience.

Education and training programs for vocal cord injection are being conducted to assist non-experts in addressing the challenges associated with accurately positioning anatomically complex objects.

To overcome the limitations of percutaneous vocal cord injection, a light-guided injection device has been developed (see Patent Document 1).

The light-guided injection device, as disclosed in Patent Document 1, comprises light source equipment and consumables, including optical fiber and injection needle. During the percutaneous vocal cord injection, an LED light source is employed to illuminate the affected area through the optical fiber, facilitating the positioning of the injection needle and enabling drug infusion to the target site simultaneously.

However, the light-guided injection device disclosed in Patent Document 1 presents the following issues.

During the vocal cord injection procedure, an assistant is necessary to support the operator in securing a clear view of the vocal cord using a nasal endoscope. However, this setup creates spatial limitations as the nasal endoscope, light source equipment, and other treatment equipment are placed in a narrow space. Consequently, performing the procedure from various angles becomes challenging.

In addition, as the length of the optical fiber increases, the energy of the light on the optical fiber from the light source gradually decreases. As a result, the brightness of the light transmitted to the affected area may appears relatively weak. To address this issue, it becomes necessary to either turn off the surrounding lights or adjust the lighting brightness of the nasal endoscope accordingly.

Furthermore, the light-guided injection device presents additional challenges, including increased production costs associated with the main elements used, potential drug loss along the drug transmitting route, and difficulties related to equipment management and maintenance.

(Patent Document 0001) (Korean Patent Registration No. 10-1699229, January 18, 2017)
US 2018/185591 A1 shows a portable light-guided injection device according to the preamble of claim 1. This known device comprises a 2-way connector part having a through-hole in a cylindrical mantle of the connector, through which hole an optical cable is fed into the interior of the connector part.
KR 2017 0051602 A shows a portable light-guided injection device that comprises a camera and has a relatively complicated design, which is detrimental to ease of maintenance and low cost in use. The device comprises a main body part that connects a syringe part and an injection needle part, and an optical cable part that transmits the light provided from a light generating part.
CN 108 245 740 A shows a portable light-guided injection device in which an optical cable part is formed integral with a needle part. This device disadvantageously requires a special needle part.
WONJAE CHA et al: "Development of a device for real-time light-guided vocal fold injection: A preliminary report", THE LARYNGOSCOPE, vol. 126, no. 4, 25 April 2016, pages 936-940 describes a non-portable light-guided injection device, without showing constructional details of an portion connecting a syringe part to a needle part.

### SUMMARY OF INVENTION

### Technical Problem

Therefore, in the related art, there exists a need for a light-guided injection device with a novel design and configuration that can address the limitations and challenges associated with existing light-guided injection devices, in particular regarding the ease of use and maintenance, and regarding the cost in use.

### Technical Solution

In order to address the aforementioned issues, the present invention introduces a portable light-guided injection device according to claim 1.

Various features of the present invention, and its advantages and effects will be understood in more detail with reference to specific embodiments below.

### Advantageous Effects

The present invention addresses the limitations of existing light-guided injection devices and offers several advantageous effects. It provides a portable light-guided injection device that is user-friendly, easy to maintain, and cost-effective while minimizing light loss and drug loss during the procedure.

### BRIEF DESCRIPTION OF THE DRAWINGS

FIGS. 1 to 6 are views illustrating a portable light-guided injection device according to an embodiment of the present invention.
FIGS. 7 and 8 are views illustrating an injection needle part according to an embodiment of the present invention.
FIG. 9 is a view illustrating a main body part according to an embodiment of the present invention.
FIGS. 10 to 12 are views illustrating a 2-way connector part according to an embodiment of the present invention.
FIG. 13 is a view illustrating the 2-way connector part and a backflow prevention fixing part coupled thereto according to an embodiment of the present invention.
FIGS. 14 to 17 are views illustrating a light generating part according to an embodiment of the present invention.
FIG. 18 is a view illustrating a main body housing according to an embodiment of the present invention.
FIGS. 19 to 20 are views illustrating a syringe part according to an embodiment of the present invention.
FIG. 21 is a view illustrating some configurations of an optical cable part and a light generating part coupled thereto according to an embodiment of the present invention.

### BEST MODE FOR INVENTION

Hereinafter, preferred embodiments will be described in detail so that those skilled in the art may easily practice the present invention with reference to the accompanying drawings. In addition, the same reference numerals are used throughout the drawings for parts having similar functions and actions.

In addition, throughout the specification, when a part is said to be 'connected' to another part, this includes not only the case where it is 'directly connected', but also the case where it is 'indirectly connected' with another element therebetween. In addition, 'including' a certain component means that other components may be further included, rather than excluding other components unless otherwise stated.

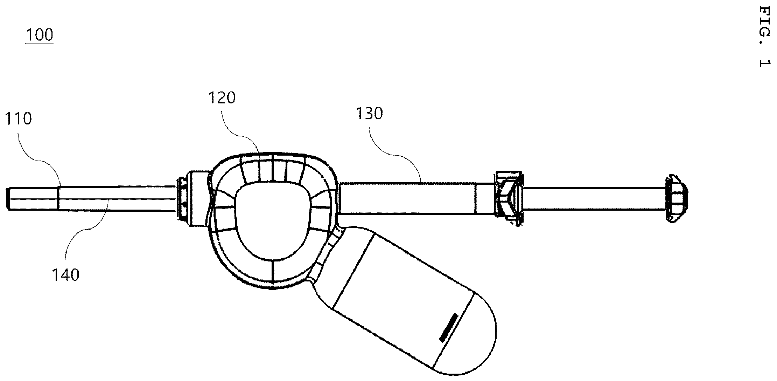

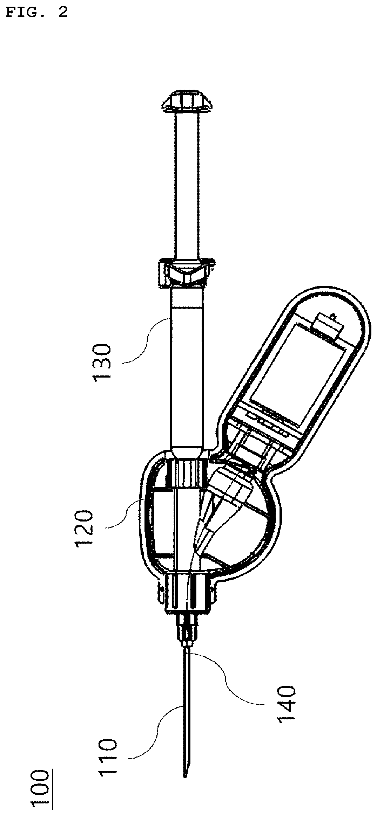

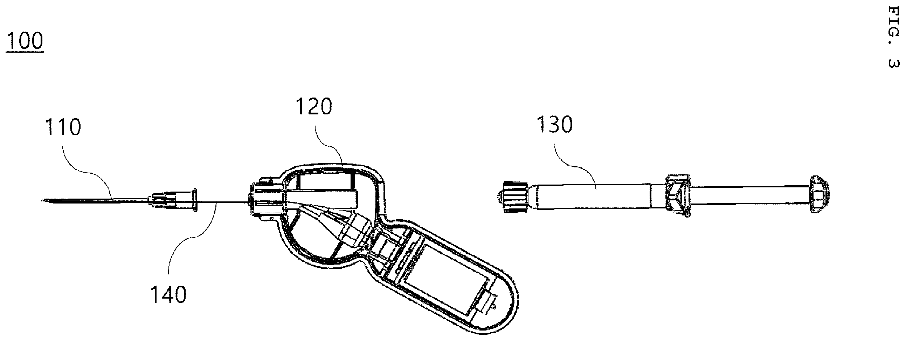

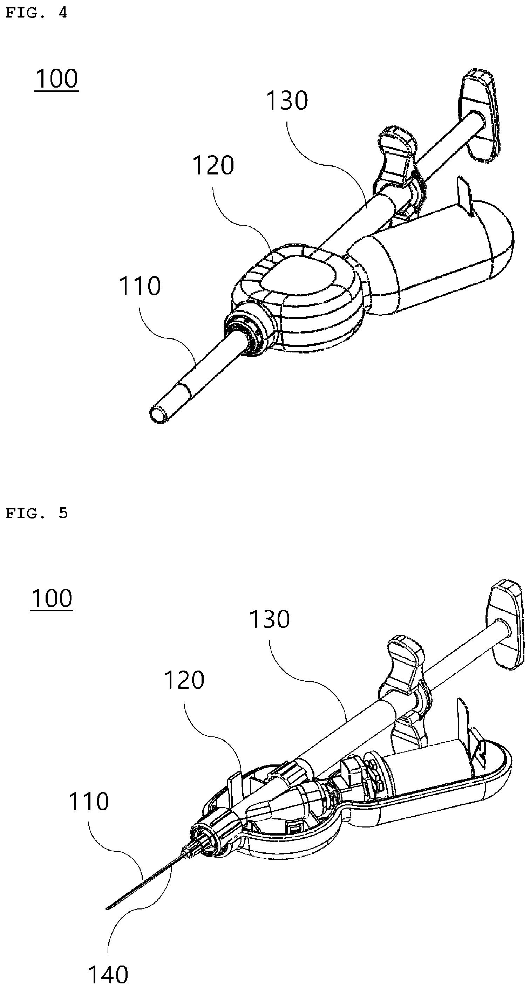

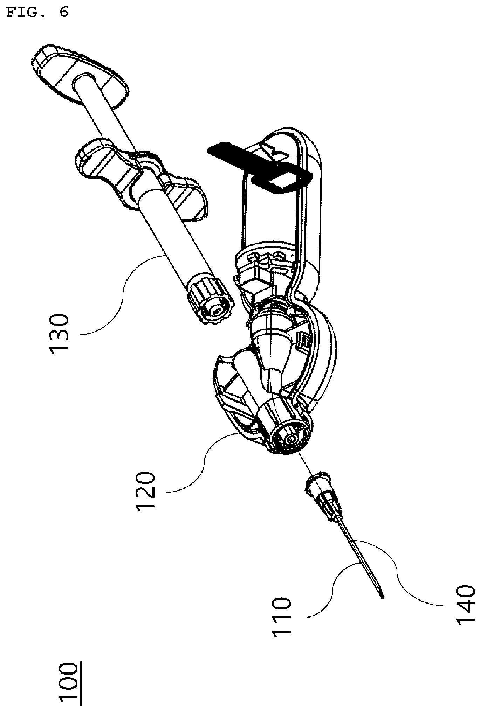

FIGS. 1 to 6 are views illustrating a portable light-guided injection device according to an embodiment of the present invention, and FIGS. 1 to 6 illustrates respectively a side view, a sectional view, an exploded sectional view, a stereoscopic view, a sectional stereoscopic view, and an exploded sectional stereoscopic view.

Referring to FIGS. 1 to 6, a portable light-guided injection device 100 according to an embodiment of the present invention is configured to include an injection needle part 110, a main body part 120, a syringe part 130, and an optical cable part 140.

Here, the injection needle part 110 invades the affected portion and injects the drug transmitted from the syringe part 130 into the body. Here, the drug to be injected may be, for example, filler, botox, steroid, silicone implant, etc., but is not necessarily limited thereto.

The main body part 120 connects the injection needle part 110 and the syringe part 130 to provide a drug transmitting path, and has a light generating part (not illustrated) configured to provide light to the affected portion, thus establishing a light transmitting path.

The syringe part 130 is for injecting the drug, and the optical cable part 140 transmits the light provided from the light generating part (not illustrated) to the affected portion.

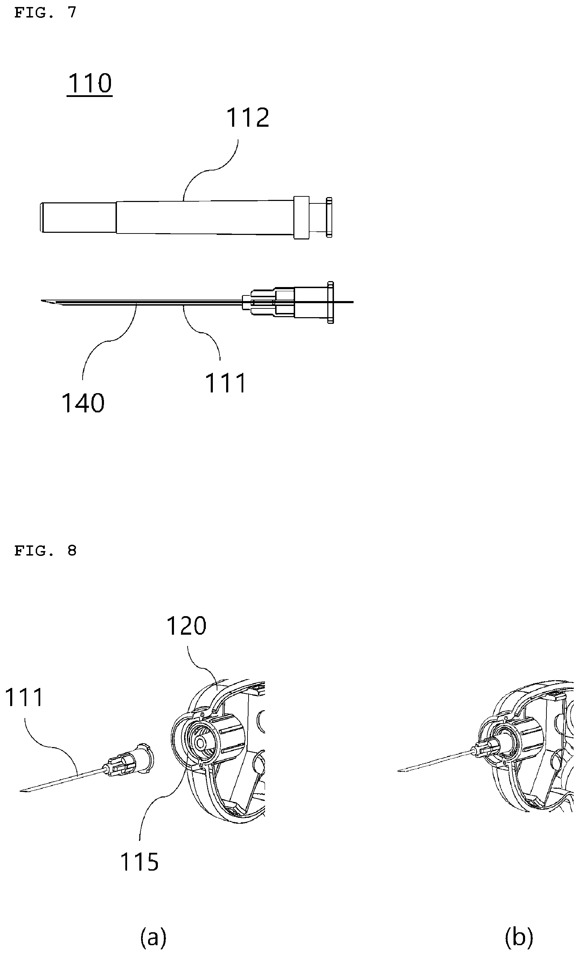

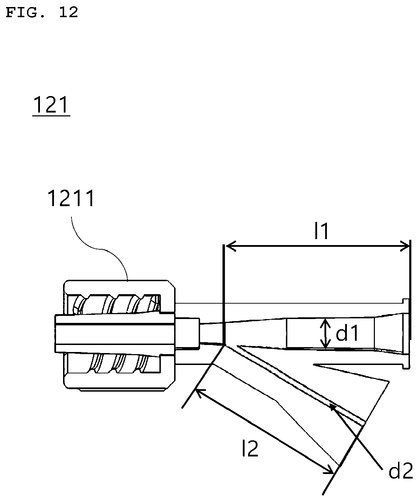

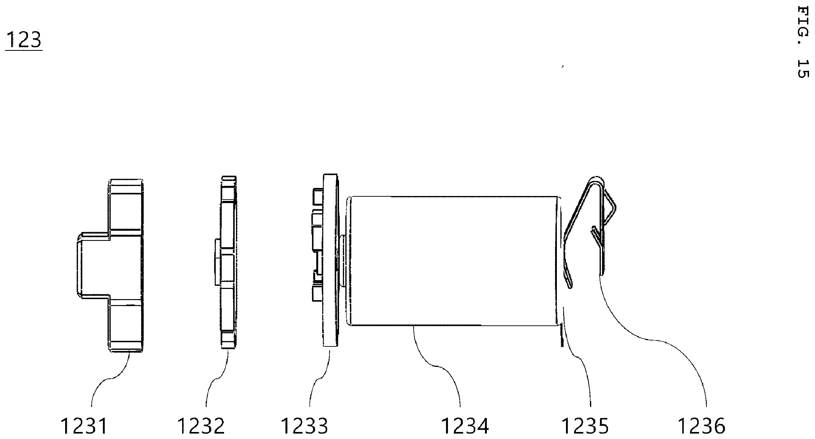

Hereinafter, each component of the portable light-guided injection device will be described in more detail with reference to FIGS. 7 to 21.

FIGS. 7 and 8 are views illustrating the injection needle part according to an embodiment of the present invention.

Referring to FIG. 7, the injection needle part 110 may include an injection needle 111 and an injection needle cap 112.

The injection needle 111 penetrates the affected portion and injects the drug into the body, and may have an outer diameter ranging from 0.305 mm to 1.270 mm and an inner diameter ranging from 0.140 mm to 0.838 mm. However, the size of the injection needle 111 is not necessarily limited thereto, and may be appropriately selected depending on the type of the drug to be injected into the body.

In addition, the optical cable part 140 to be described later is inserted through the hollow interior of the injection needle 111, through which light is transmitted to the affected portion.

The injection needle cap 112 is for protecting the injection needle 111 and preventing stabbing, and is coupled to the injection needle 111 to cover the outside of the injection needle 111, and may be removed from the injection needle 111 during the operating procedure.

Referring to FIG. 8, as illustrated in (a), the injection needle part 110 and the main body part 120 may be separated from each other, and the injection needle part 110 and the main body part 120 may be connected to each other by a luer lock 115 (see (b)).

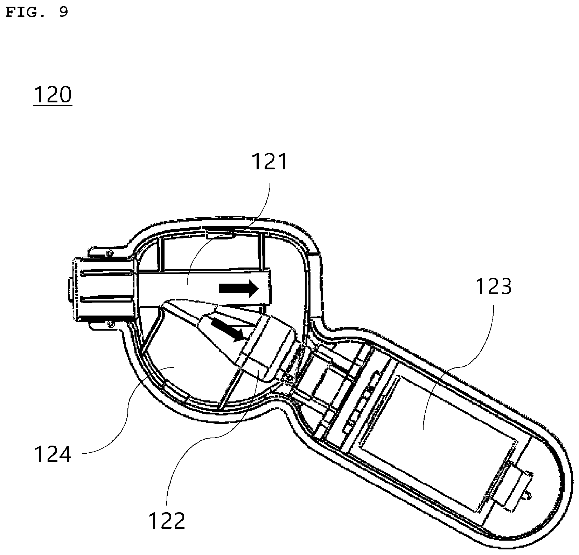

FIG. 9 is a view illustrating the main body part according to an embodiment of the present invention.

Referring to FIG. 9, the main body part 120 includes a 2-way connector part 121, a backflow prevention fixing part 122, a light generating part 123, and a main body housing 124.

Here, one end of the 2-way connector part 121 is connected to the injection needle part 110, a first direction (upper arrow in FIG. 9) of the other end is connected to the syringe part 130, and a second direction (lower arrow in FIG. 9) of the other end is connected to the light generating part 123 to provide both the drug transmitting path and the light transmitting path.

The backflow prevention fixing part 122 secures the optical cable part 140 and simultaneously prevents the drug injected from the first direction (drug injecting part) from flowing backward in the second direction (light source transmitting part).

The light generating part 123 generates the light to be provided to the affected portion.

The main body housing 124 forms an inner space that accommodates the 2-way connector part 121, the backflow prevention fixing part 122, and the light generating part 123.

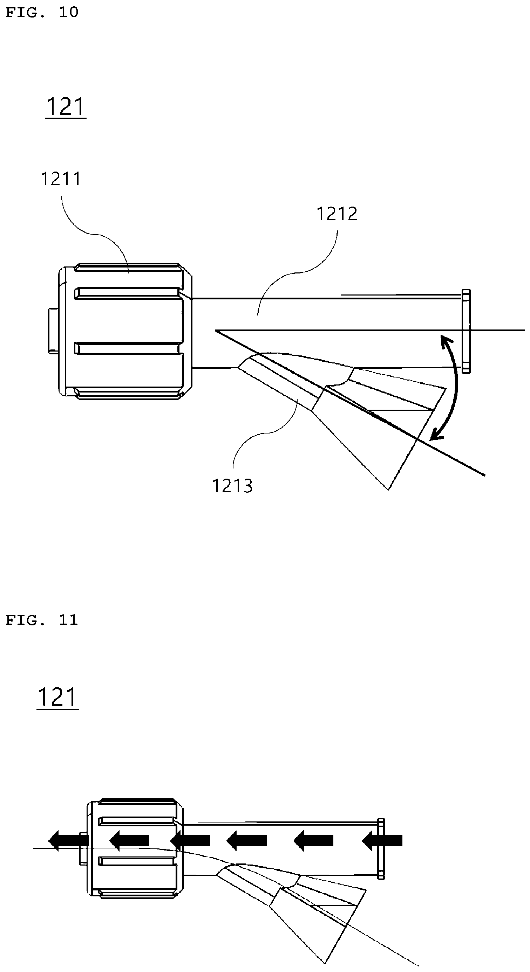

FIGS. 10 to 12 are views illustrating the 2-way connector part according to an embodiment of the present invention.

Referring to FIGS. 10 to 12, the 2-way connector part 121 includes a first connector part 1211, a drug injecting part 1212, and a light source transmitting part 1213.

Here, the first connector part 1211 is connected to the injection needle part 110 and may be a luer lock type connector.

The drug injecting part 1212 accommodates the drug that is injected from the syringe part 130, providing the drug transmitting path (arrow in FIG. 11), and the light source transmitting part 1213 accommodates the optical cable part 140, which is inserted through the backflow prevention fixing part 122, providing the light transmitting path (solid line in FIG. 11).

In this case, the drug injecting part 1212 and the light source transmitting part 1213are designed to form an acute angle (ie, an angle smaller than 90°) with each other, preferably the angle formed by the drug injecting part 1212 and the light source transmitting part 1213 may ranges from 20° to 80°. By doing so, it is possible to facilitate insertion of the optical cable part 140 and to prevent bending of the optical cable part 140.

In addition, to minimize drug loss, the length l1 of the drug injecting part 1212 may be designed to be shorter than the length l2 of the light source transmitting part 1213.

Moreover, by designing the inner diameter d2 of the light source transmitting part 1213 smaller than the inner diameter d1 of the drug injecting part 1212, it is possible to prevent drug backflow and minimize drug loss during drug injection. According to an embodiment, the inner diameter d2 of the light source transmitting part 1213 can be matched to the outer diameter of the optical cable part 140 that is inserted into the light source transmitting part 1213.

The aforementioned 2-way connector part 121 may be made of an opaque material to prevent light scattering.

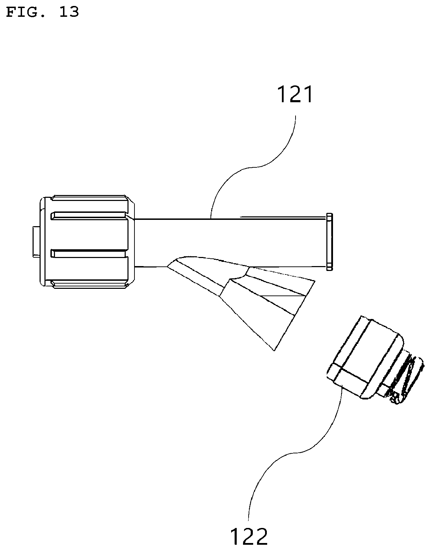

FIG. 13 is a view illustrating the 2-way connector part and the backflow prevention fixing part coupled thereto according to an embodiment of the present invention.

Referring to FIG. 13, the backflow prevention fixing part 122 is coupled to the end of the light source transmitting part 1213 of the 2-way connector part 121 to secure the optical cable part 140 inserted into the light source transmitting part 1213 and prevent the drug backflow.

The backflow prevention fixing part 122 may be configured, for example, in various forms such as such as a STEM swabable silicone valve, a silicone sealing, a fixing cap, and a directly fixing method using an adhesive like epoxy.

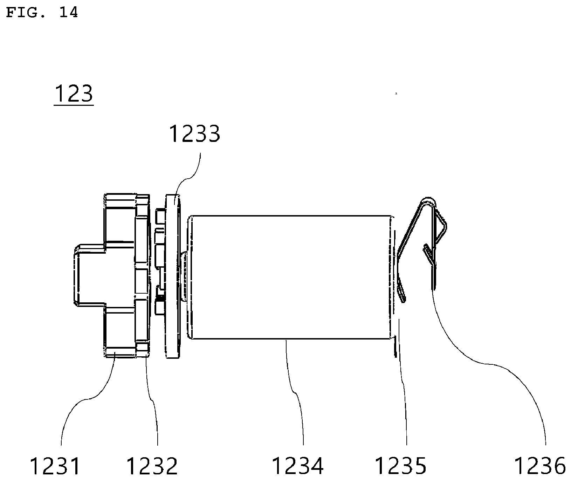

In addition, the backflow prevention fixing part 122 may be integrated with a light source cap part (1231 in FIG. 14) to be described later.

FIGS. 14 to 17 are views illustrating the light generating part according to an embodiment of the present invention.

Referring to FIGS. 14 to 17, the light generating part 123 may include the light source cap part 1231, a light source 1232, a heat sink 1232', a control board 1233, a power supply part 1234, a switch part 1235, and a contact plate 1236.

The light source cap part 1231 may be coupled to the light source 1232 to prevent scattering of the light, and may ensure proper alignment and fixation of the centers of the light source 1232 and the optical cable part 140. The light source cap part 1231 may be, for example, a connector such as SMA, FC, and ST, or a ceramic connector, but is not necessarily limited thereto.

The light source 1232 generates light when power is applied. The light source 1232 may be, for example, a laser diode, or LED, but is not necessarily limited thereto.

In addition, the control board 1233 and the heat sink 1232' may be selectively provided according to the type of the light source 1232 used. Here, the control board 1233 is provided for adjusting the applied voltage, while the heat sink 1232' is provided for dissipating the heat generated by the light source 1232, and the control board 1233 and the heat sink 1232' may be implemented, for example, using a PCB board.

The power supply part 1234 supplies power to the light source 1232.

The switch part 1235 is provided for controlling power supply from the power supply part 1234.

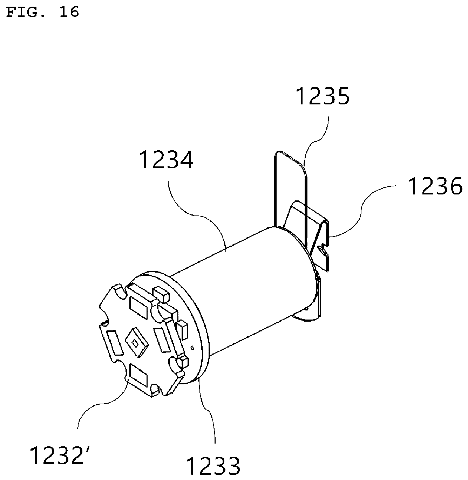

According to an embodiment, as illustrated in FIG. 16, the switch part 1235 is designed as a thin plate located between the power supply part 1234 and the contact plate 1236 to establish or break the connection between the power supply part 1234 and the contact plate 1236.

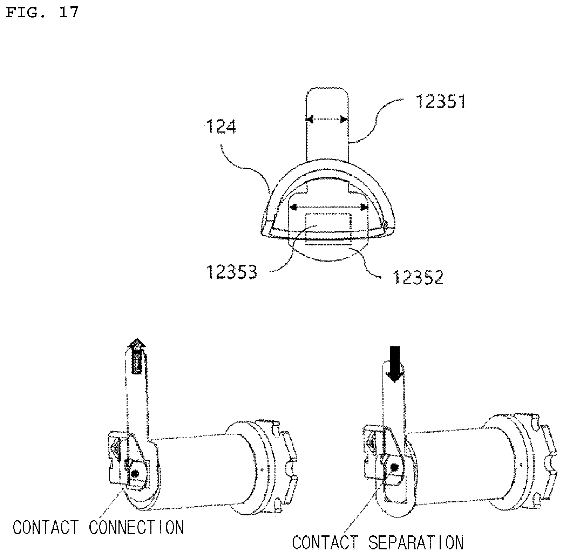

Specifically, as illustrated in FIG. 17, the switch part 1235 may include a handle part 12351, a head part 12352, and a connection hole 12353 situated within the head part 12352.

The handle part 12351 is a portion that protrudes from the main body housing 124 and may be gripped to push and pull the switch part 1235.

The head part 12352 is a portion that extends from the handle part 12351 to be integrally formed, and is inserted into the main body housing 124 to be positioned between the power supply part 1234 and the contact plate 1236.

The connection hole 12353 is formed in a specific area of the head part 12352, and as the switch part 1235 moves, the connection hole 12353 enables the power supply part 1234 and the contact plate 1236 to establish a connection. Accordingly, the power supply part 1234 supply power to the light source 1232, resulting in the generation of light.

A switch hole (not illustrated) is incorporated into the main body housing 124 so that the handle part 12351 integrally formed with the head part 12352 extends outside of the main body housing 124 through the switch hole (not illustrated).

The width of the handle part 12351 may be designed to be narrower than the width of the head part 12352, the width of the handle part 12351 may be narrower than the width of the switch hole (not illustrated), and the width of the head part 12352 may be made wider than the width of the switch hole (not illustrated). In this case, since the switch part 1235 is not separated from the main body housing 124 to the outside, it is possible to prevent loss thereof.

The configuration of the switch part 1235 described above with reference to FIG. 17 is only an example, and the switch part 1235 may be modified in various forms.

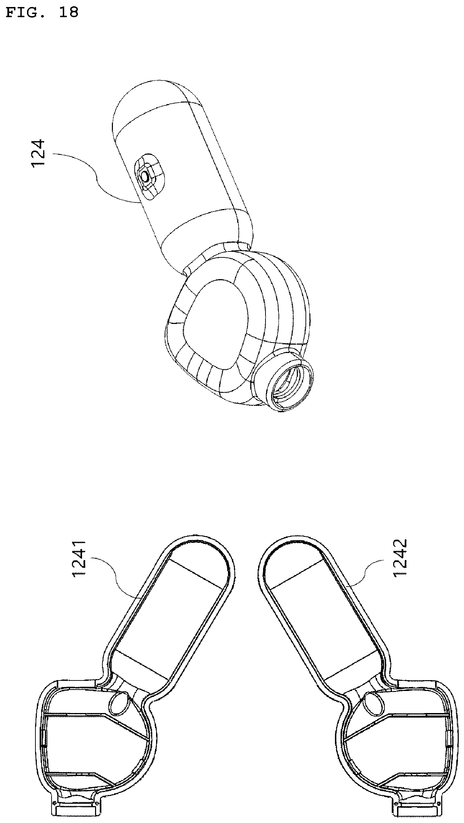

FIG. 18 is a view illustrating a main body housing according to an embodiment of the present invention.

Referring to FIG. 18, the main body housing 124 is composed of a first housing 1241 covering one surface of the main body part and a second housing 1242 covering the other surface of the main body part, and the first housing 1241 and the second housing 1242 are coupled together to form a space therein to accommodate the 2-way connector part 121, the backflow prevention fixing part 122, and the light generating part 123.

In addition, as described above with reference to FIG. 17, the switch hole (not illustrated) may be formed in a portion corresponding to the area where the power supply part 1234 and the contact plate 1236 are connected to each other within the main body housing 124.

Furthermore, the main body housing 124 is designed with an ergonomic shape to ensure comfortable and convenient handling for users when the device is used in its portable form.

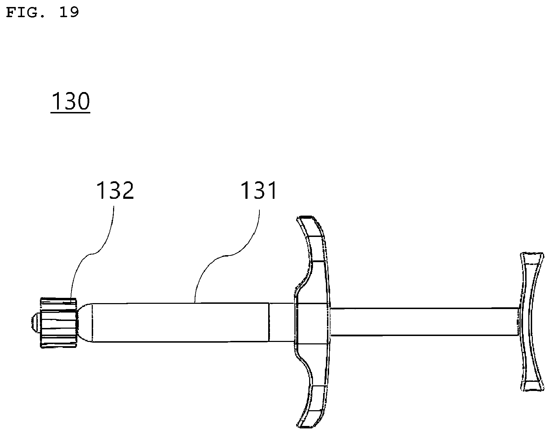

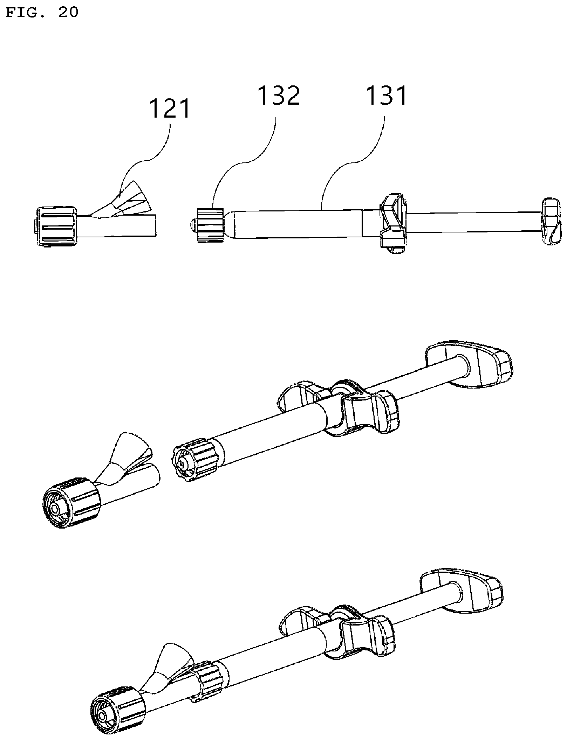

FIGS. 19 and 20 are views illustrating the syringe part according to an embodiment of the present invention.

Referring to FIGS. 19 and 20, the syringe part 130 may include a syringe 131 and a second connector part 132.

Here, the second connector part 132 connects the syringe 131 and the 2-way connector part 121, and may be a luer lock type connector.

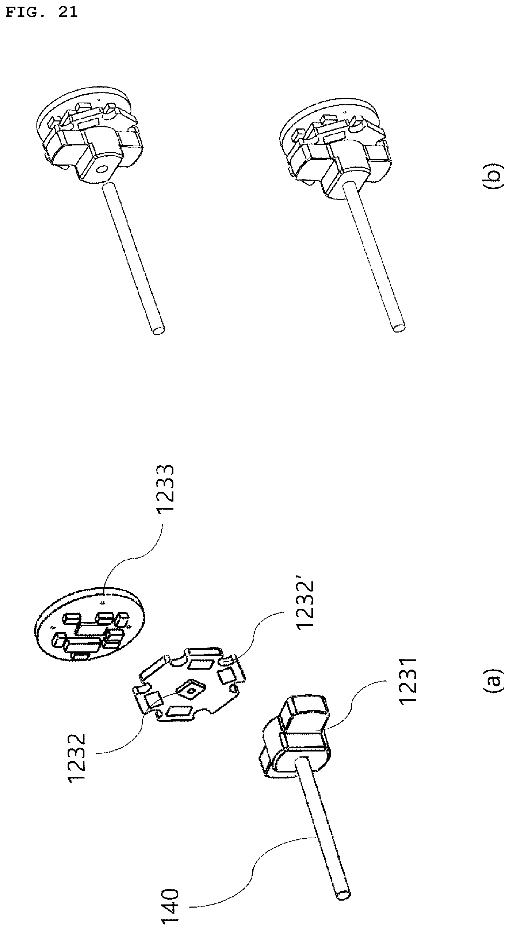

FIG. 21 is a view illustrating some configurations of the optical cable part and the light generating part coupled thereto according to an embodiment of the present invention.

Referring to FIG. 21, the optical cable part 140 serves as a medium for transmitting light generated from the light source 1232, and may be implemented using various materials, for example, PMMA (plastic-based), silica (glass-based) optical fibers, or the like.

The optical cable part 140 may be connected to or detached from the light source cap part 1231. For example, the optical cable part 140 may be fixed to the light source cap part 1231 using adhesive or may be connected thereto connectors such as SMA or FC.

The diameter of the optical cable part 140 may range from 10 µm to 200 µm, but is not necessarily limited thereto.

In addition, the optical cable part 140 may be inserted into the injection needle 111 through the backflow prevention fixing part 122, the light source transmitting part 1213, and the first connector part 1211 to transmit light to the affected portion.

The present invention is not limited by the foregoing embodiments and accompanying drawings.

## Claims

1. A portable light-guided injection device (100) comprising:
a syringe part (130) that injects drug;
an injection needle part (110) that invades an affected portion and injects the drug into the body;
a main body part (120) that connects the syringe part (130) and the injection needle part (110) to provide a drug transmitting path and has a light generating part (123) configured to provide light to the affected portion; and
an optical cable part (140) that transmits the light provided from the light generating part (123) to the affected portion,
wherein the main body part (120) includes
a 2-way connector part (121) of which one end is connected to the injection needle part (110) and the other end is connected to the syringe part (130) to provide the drug transmitting path,
wherein the 2-way connector part (121) includes
a first connector part (1211) that is connected to the injection needle part (110), and
a drug injecting part (1212) that accommodates the drug injected from the syringe part (130) and provides the drug transmitting path,
**characterized in that**
- a first direction of the other end of the 2-way connector part (121) is connected to the syringe part (130) and a second direction of the other end of the 2-way connector part (121) is connected to the light generating part (123) to provide the drug transmitting path and a light transmitting path,
- the main body part (120) further includes a backflow prevention fixing part (122) that is coupled to the end of the 2-way connector part (121) in the second direction to fix the optical cable part (140) and prevent a backflow of the drug, and a main body housing (124) that accommodates the light generating part (123), the 2-way connector part (121), and the backflow prevention fixing part (122) in an inner space,
- the 2-way connector part (121) further includes a light source transmitting part (1213) that accommodates the optical cable part (140) inserted through the backflow prevention fixing part (122) and provides a light transmitting part, and
- the drug injecting part (1212) and the light source transmitting part (1213) are formed to form an acute angle with each other.

2. The portable light-guided injection device (100) of claim 1, wherein a length (l1) of the drug injecting part (1212) is formed shorter than a length (l2) of the light source transmitting part (1213).

3. The portable light-guided injection device (100) of claim 1, wherein an inner diameter (d2) of the light source transmitting part (1213) is formed smaller than an inner diameter (d1) of the drug injecting part (1212).

4. The portable light-guided injection device (100) of claim 1, wherein the 2-way connector part (121) is formed of a material of opaque color.

5. The portable light-guided injection device (100) of claim 1, wherein the light generating part (123) includes
a light source (1232) that generates light;
a power supply part (1234) that applies power to the light source (1232);
a switch part (1235) that controls power supply by the power supply part (1234); and
a light source cap part (1231) that is coupled to the light source (1232) to prevent scattering of the light source (1232) and aligns and fixes centers of the light source (1232) and the optical cable part (140).

6. The portable light-guided injection device (100) of claim 5, wherein the light generating part (123) further includes at least one of
a control board (1233) that adjusts a voltage applied to the light source (1232); and
heat sinks (1232') that dissipate heat generated in the light source (1232).

7. The portable light-guided injection device (100) of claim 5, wherein the switch part (1235) is formed in a plate shape located between the power supply part (1234) and a contact plate (1236) to connect or disconnect between the power supply part (1234) and the contact plate (1236).

8. The portable light-guided injection (100) device of claim 7, wherein the switch part (1235) includes
a handle part (12351) that protrudes to the outside of the main body housing (124) and is configured to hold the switch portion to push and pull the switch portion;
a head part (12352) that extends from the handle part (12351) and is integrally formed, and inserted into the main body housing (124) to be positioned between the power source part and the contact plate (1236); and
a connection hole (12353) that is formed in a partial region of the head part (12352), and
the power supply part (1234) and the contact plate (1236) are connected to each other through the connection hole (12353) as the switch part (1235) moves.

9. The portable light-guided injection device (100) of claim 8, wherein a switch hole is formed in the main body housing (124) so that the handle part (12351) protrudes to the outside of the main body housing (124) through the switch hole, and
a width of the handle part (12351) is formed smaller than a width of the head part (12352), the width of the handle part (12351) is formed smaller than a width of the switch hole, and the width of the head part (12352) is formed larger than the width of the switch hole.

10. The portable light-guided injection device (100) of claim 1, wherein the syringe part (130) includes
a syringe (131); and
a second connector part (132) that connects the syringe (131) and the 2-way connector part (121).

11. The portable light-guided injection device (100) of claim 5, wherein the optical cable part (140) is coupled to or separated from the light source cap part (1231), and
the optical cable part (140) is inserted into the injection needle (111) through the backflow prevention fixing part (122), the light source transmitting part (1213), and the first connector part (1211) to transmit light to the affected portion.

## Patentansprüche

1. Tragbare lichtgesteuerte Injektionsvorrichtung (100), umfassend:
ein Spitzenteil (130), das ein Medikament injiziert;
ein Injektionsnadelteil (110), das in einen betroffenen Bereich eindringt und das Medikament in den Körper injiziert;
ein Hauptkörperteil (120), das das Spritzenteil (130) und das Injektionsnadelteil (110) verbindet, um einen Medikamentenübertragungsweg bereitzustellen, und ein Lichterzeugungsteil (123) aufweist, das dazu konfiguriert ist, Licht an den betroffenen Bereich abzugeben; und
ein optisches Kabelteil (140), das das vom Lichterzeugungsteil (123) abgegebene Licht an den betroffenen Bereich überträgt,
wobei das Hauptkörperteil (120) umfasst:
ein 2-Wege-Verbindungsteil (121), von dem ein Ende mit dem Injektionsnadelteil (110) und das andere Ende mit dem Spritzenteil (130) verbunden ist, um den Medikamentenübertragungsweg bereitzustellen,
wobei das 2-Wege-Verbindungsteil (121) umfasst:
ein erstes Verbindungsteil (1211), das mit dem Injektionsnadelteil (110) verbunden ist, und
ein Medikamenteninjektionsteil (1212), das das aus dem Spritzenteil (130) injizierte Medikament aufnimmt und den Medikamentenübertragungsweg bereitstellt,
**dadurch gekennzeichnet, dass**
- eine erste Richtung des anderen Endes des 2-Wege-Verbindungsteils (121) mit dem Spritzenteil (130) verbunden ist und eine zweite Richtung des anderen Endes des 2-Wege-Verbindungsteils (121) mit dem Lichterzeugungsteil (123) verbunden ist, um den Medikamentenübertragungsweg und einen Lichtübertragungsweg bereitzustellen,
- das Hauptkörperteil (120) ferner ein Rückflussverhinderungs-Befestigungsteil (122), das mit dem Ende des 2-Wege-Verbindungsteils (121) in der zweiten Richtung verbunden ist, um das optische Kabelteil (140) zu befestigen und einen Rückfluss des Medikaments zu verhindern, sowie ein Hauptkörpergehäuse (124) umfasst, das das Lichterzeugungsteil (123), das 2-Wege-Verbindungsteil (121) und das Rückflussverhinderungs-Befestigungsteil (122) in einem Innenraum aufnimmt,
- das 2-Wege-Verbindungsteil (121) ferner ein Lichtquellenübertragungsteil (1213) umfasst, das das durch das Rückflussverhinderungs-Befestigungsteil (122) eingeführte optische Kabelteil (140) aufnimmt und einen Lichtübertragungsweg bereitstellt, und
- das Medikamenteninjektionsteil (1212) und das Lichtquellenübertragungsteil (1213) derart ausgebildet sind, dass sie einen spitzen Winkel miteinander bilden.

2. Tragbare lichtgesteuerte Injektionsvorrichtung (100) nach Anspruch 1, wobei eine Länge (l1) des Medikamenteninjektionsteils (1212) kürzer ausgebildet ist als eine Länge (12) des Lichtquellenübertragungsteils (1213).

3. Tragbare lichtgesteuerte Injektionsvorrichtung (100) nach Anspruch 1, wobei ein Innendurchmesser (d2) des Lichtquellenübertragungsteils (1213) kleiner ausgebildet ist als ein Innendurchmesser (d1) des Medikamenteninjektionsteils (1212).

4. Tragbare lichtgesteuerte Injektionsvorrichtung (100) nach Anspruch 1, wobei das 2-Wege-Verbindungsteil (121) aus einem Material mit opaker Farbe ausgebildet ist.

5. Tragbare lichtgesteuerte Injektionsvorrichtung (100) nach Anspruch 1, wobei das Lichterzeugungsteil (123) umfasst:
eine Lichtquelle (1232), die Licht erzeugt;
ein Stromversorgungsteil (1234), das die Lichtquelle (1232) mit Strom versorgt;
ein Schaltteil (1235), das die Stromversorgung durch das Stromversorgungsteil (1234) steuert; und
ein Lichtquellenabdeckteil (1231), das mit der Lichtquelle (1232) gekoppelt ist, um eine Streuung des Lichts der Lichtquelle (1232) zu verhindern und die Mittelpunkte der Lichtquelle (1232) und des optischen Kabelteils (140) auszurichten und zu fixieren.

6. Tragbare lichtgesteuerte Injektionsvorrichtung (100) nach Anspruch 5, wobei das Lichterzeugungsteil (123) ferner mindestens eines der folgenden Elemente umfasst:
eine Steuerplatine (1233), die eine an die Lichtquelle (1232) angelegte Spannung einstellt; und
Kühlkörper (1232'), die in der Lichtquelle (1232) erzeugte Wärme ableiten.

7. Tragbare lichtgesteuerte Injektionsvorrichtung (100) nach Anspruch 5, wobei das Schaltteil (1235) in Form einer Platte ausgebildet ist, die sich zwischen dem Stromversorgungsteil (1234) und einer Kontaktplatte (1236) befindet, um eine Verbindung zwischen dem Stromversorgungsteil (1234) und der Kontaktplatte (1236) herzustellen oder zu unterbrechen.

8. Tragbare lichtgesteuerte Injektionsvorrichtung (100) nach Anspruch 7, wobei das Schaltteil (1235) umfasst:
ein Griffteil (12351), das aus dem Hauptkörpergehäuse (124) nach außen ragt und dazu konfiguriert ist, den Schaltbereich zu halten, um den Schaltbereich zu drücken und zu ziehen;
ein Kopfteil (12352), das sich vom Griffteil (12351) erstreckt und integral ausgebildet ist und in das Hauptkörpergehäuse (124) eingeführt ist, um zwischen dem Stromversorgungsteil und der Kontaktplatte (1236) positioniert zu werden; und
ein Verbindungsloch (12353), das in einem Teilbereich des Kopfteils (12352) ausgebildet ist, und
wobei das Stromversorgungsteil (1234) und die Kontaktplatte (1236) durch das Verbindungsloch (12353) miteinander verbunden werden, wenn sich das Schaltteil (1235) bewegt.

9. Tragbare lichtgesteuerte Injektionsvorrichtung (100) nach Anspruch 8, wobei im Hauptkörpergehäuse (124) eine Schalteröffnung ausgebildet ist, derart dass das Griffteil (12351) durch die Schalteröffnung aus dem Hauptkörpergehäuse (124) nach außen ragt, und
eine Breite des Griffteils (12351) kleiner als eine Breite des Kopfteils (12352) ausgebildet ist, die Breite des Griffteils (12351) kleiner als eine Breite der Schalteröffnung ausgebildet ist und die Breite des Kopfteils (12352) größer als die Breite der Schalteröffnung ausgebildet ist.

10. Tragbare lichtgesteuerte Injektionsvorrichtung (100) nach Anspruch 1, wobei das Spritzenteil (130) umfasst:
eine Spritze (131); und
ein zweites Verbindungsteil (132), das die Spritze (131) und das 2-Wege-Verbindungsteil (121) verbindet.

11. Tragbare lichtgesteuerte Injektionsvorrichtung (100) nach Anspruch 5, wobei das optische Kabelteil (140) mit dem Lichtquellenabdeckteil (1231) verbunden oder von diesem getrennt ist, und
das optische Kabelteil (140) durch das Rückflussverhinderungs-Befestigungsteil (122), das Lichtquellenübertragungsteil (1213) und das erste Verbindungsteil (1211) in die Injektionsnadel (111) eingeführt wird, um Licht an den betroffenen Bereich zu übertragen.

## Revendications

1. Dispositif d'injection portatif guidé par la lumière (100) comprenant :
une partie seringue (130) qui injecte un médicament ;
une partie aiguille d'injection (110) qui pénètre une région affectée et injecte le médicament dans le corps ;
une partie de corps principal (120) qui relie la partie seringue (130) et la partie aiguille d'injection (110) pour fournir un chemin de transmission du médicament et qui comporte une partie de génération de lumière (123) configurée pour fournir lumière à la région affectée ; et
une partie de câble optique (140) qui transmet la lumière fournie par la partie de génération de lumière (123) à la région affectée,
dans lequel la partie de corps principal (120) comprend
une partie de connecteur à deux voies (121) dont une extrémité est reliée à la partie aiguille d'injection (110) et l'autre extrémité est reliée à la partie seringue (130) afin de former le chemin de transmission du médicament,
dans lequel la partie de connecteur à deux voies (121) comprend
une première partie de raccordement (1211) qui est reliée à la partie aiguille d'injection (110), et
une partie d'injection de médicament (1212) qui reçoit le médicament injecté depuis la partie seringue (130) et forme le chemin de transmission du médicament,
**caractérisé en ce que**
- une première direction de l'autre extrémité de la partie de connecteur à deux voies (121) est reliée à la partie seringue (130) et une deuxième direction de l'autre extrémité de la partie de connecteur à deux voies (121) est reliée à la partie de génération de lumière (123) afin de former le chemin de transmission du médicament et un chemin de transmission de lumière,
- la partie de corps principal (120) comprend en outre une partie de fixation anti-refoulement (122) qui est couplée à l'extrémité de la partie de connecteur à deux voies (121) dans la deuxième direction pour fixer la partie de câble optique (140) et empêcher un refoulement du médicament, et un boîtier de corps principal (124) qui loge la partie de génération de lumière (123), la partie de connecteur à deux voies (121) et la partie de fixation anti-refoulement (122) dans un espace intérieur,
- la partie de connecteur à deux voies (121) comprend en outre une partie de transmission de source lumineuse (1213) qui loge la partie de câble optique (140) insérée à travers la partie de fixation anti-refoulement (122) et fournit une voie de transmission de lumière, et
- la partie d'injection de médicament (1212) et la partie de transmission de source lumineuse (1213) sont formées de manière à former un angle aigu l'une par rapport à l'autre.

2. Dispositif d'injection portatif guidé par la lumière (100) selon la revendication 1, dans lequel une longueur (l1) de la partie d'injection de médicament (1212) est formée plus courte qu'une longueur (12) de la partie de transmission de source lumineuse (1213).

3. Dispositif d'injection portatif guidé par la lumière (100) selon la revendication 1, dans lequel un diamètre intérieur (d2) de la partie de transmission de source lumineuse (1213) est plus petit qu'un diamètre intérieur (d1) de la partie d'injection de médicament (1212).

4. Dispositif d'injection portatif guidé par la lumière (100) selon la revendication 1, dans lequel la partie de connecteur à deux voies (121) est formée d'un matériau de couleur opaque.

5. Dispositif d'injection portatif guidé par la lumière (100) selon la revendication 1, dans lequel la partie de génération de lumière (123) comprend
une source de lumière (1232) qui génère de la lumière ;
une partie d'alimentation à énergie (1234) qui alimente la source de lumière (1232) ;
une partie de commutation (1235) qui commande l'alimentation à énergie par la partie d'alimentation à énergie (1234) ; et
une partie de capuchon de source lumineuse (1231) qui est couplée à la source de lumière (1232) pour empêcher la diffusion de la source de lumière (1232) et qui aligne et fixe les centres de la source de lumière (1232) et de la partie de câble optique (140).

6. Dispositif d'injection portatif guidé par la lumière (100) selon la revendication 5, dans lequel la partie de génération de lumière (123) comprend en outre au moins l'un parmi :
une platine de commande (1233) qui régule une tension appliquée à la source de lumière (1232) ; et
des dissipateurs de chaleur (1232') qui dissipent une chaleur générée dans la source de lumière (1232).

7. Dispositif d'injection portatif guidé par la lumière (100) selon la revendication 5, dans lequel la partie de commutation (1235) est réalisée en forme d'une plaque située entre la partie d'alimentation à énergie (1234) et une plaque de contact (1236) afin de connecter ou de déconnecter la partie d'alimentation à énergie (1234) et la plaque de contact (1236).

8. Dispositif d'injection portatif guidé par la lumière (100) selon la revendication 7, dans lequel la partie de commutation (1235) comprend :
une partie poignée (12351) qui fait saillie vers l'extérieur du boîtier de corps principal (124) et est configurée pour tenir la partie de commutation afin de pousser et de tirer la partie de commutation ;
une partie de tête (12352) qui s'étend à partir de la partie poignée (12351) et est formée intégralement, et qui est insérée dans le boîtier de corps principal (124) pour être positionnée entre la partie d'alimentation à énergie et la plaque de contact (1236) ; et
un trou de connexion (12353) formé dans une région partielle de la partie tête (12352), et
la partie d'alimentation à énergie (1234) et la plaque de contact (1236) sont reliées l'une à l'autre à travers le trou de connexion (12353) lorsque la partie de commutation (1235) se déplace.

9. Dispositif d'injection portatif guidé par la lumière (100) selon la revendication 8, dans lequel un trou de commutation est formé dans le boîtier de corps principal (124) de telle sorte que la partie poignée (12351) dépasse à l'extérieur du boîtier de corps principal (124) à travers ledit trou de commutation, et
une largeur de la partie poignée (12351) est formée plus petite qu'une largeur de la partie tête (12352), la largeur de la partie poignée (12351) est formée plus petite qu'une largeur de trous de commutation, et la largeur de la partie tête (12352) est formée plus grande que la largeur du trou de commutation.

10. Dispositif d'injection portatif guidé par la lumière (100) selon la revendication 1, dans lequel la partie seringue (130) comprend
une seringue (131) ; et
une deuxième partie de connecteur (132) qui relie la seringue (131) et la partie de connecteur à deux voies (121).

11. Dispositif d'injection portatif guidé par la lumière (100) selon la revendication 5, dans lequel la partie de câble optique (140) est couplée à la partie de capuchon de source lumineuse (1231) ou séparée de celle-ci, et
la partie de câble optique (140) est insérée dans l'aiguille d'injection (111) à travers la partie de fixation anti-refoulement (122), la partie de transmission de source lumineuse (1213) et la première partie de connecteur (1211) afin de transmettre lumière à la région affectée.
